# EUROPEAN PATENT APPLICATION

(11) **EP 0 855 188 A2**
(43) Date of publication of application: **29.07.1998**
(21) Application number: 98300489.6
(22) Date of filing: 23.01.1998
(51) Int. Cl.: A61L 2/18, A01N 59/00

(54) **Contact lens solution**

(30) Priority: 23.01.1997 GB 9701338
(71) Applicant: Waverley Pharmaceutical Limited, Runcorn, Cheshire, WA7 1QE (GB)
(72) Inventor: Holloway, John, Manor Park, Runcorn, Cheshire WA7 1TG (GB)
(74) Representative: Browne, Robin Forsythe, Dr.

(57) **Abstract**

An hydrogen peroxide solution for sterilising contact lenses, characterised in that the solution further contains an agent such as glycerol serving to reduce the overall rate of hydrogen peroxide decomposition when in the presence of a suitable catalyst, thereby prolonging the effective life of the solution and providing greater comfort to the wearer.

## Description

The present invention relates to solutions of hydrogen peroxide used in the sterilisation of contact lenses.

Contact lenses have proven to be extremely useful for people with sight defects who either do not wish to wear spectacles, or cannot wear them. Although they are very convenient, one problem with contact lenses is that they have to be cleaned. Disposable contact lenses can be thrown away after one day's use and do not suffer this problem, but this requires the wearer to, first, maintain a supply of disposable lenses and, second, disposable lenses may not be available to everybody, depending on the reasons for wearing contact lenses in the first place.

Hard contact lenses can be boil washed. This can be inconvenient and, in addition, many people cannot wear hard lenses owing to irritation of the eye.

Thus, the most popular form of contact lens is the soft, gas-permeable contact lens, which is not so likely to irritate the eye, and which can be worn for months, or even years, subject to regular cleaning.

Cleaning of soft contact lenses is particularly important, as the porous nature of the lens can permit invasion by such matter as dirt and micro-organisms. Thus, after each period of use, it is recommended to wash and sterilise the lenses, and subsequently to keep them in a buffer solution until they are required once more.

Sterilisation of contact lenses is usually involves exposure to a solution of hydrogen peroxide in order to kill any undesirable micro-organisms As the eye is sensitive to hydrogen peroxide, the contact lenses must be washed or otherwise freed from hydrogen peroxide before use. It is still commonplace for lenses first to be exposed to a solution of hydrogen peroxide, typically after a detergent wash and rinse, and, after allowing the lenses to stand for a period of time, draining off the lenses, neutralising any remaining hydrogen peroxide by decomposition to oxygen and water, and then rinsing the lenses with saline before use.

Recently, one-step sterilisation has been introduced, where the lenses, optionally previously washed and rinsed, are immersed in a solution of hydrogen peroxide. A suitable catalyst, typically a platinum salt, is present in the lens container, and this serves to completely decompose the hydrogen peroxide within about 20 minutes. The catalyst conveniently takes the form of a platinum oxide disc, which catalyses the decomposition of hydrogen peroxide to water and oxygen gas while sterilisation proceeds, sterilisation being effected by undecomposed hydrogen peroxide.

One problem with platinum oxide-catalysed hydrogen peroxide lens cleaning procedures is that some micro-organisms, such as Acanthamoeba, take longer than 20 minutes to be killed by hydrogen peroxide. Thus, it will be appreciated that such sterilisation procedures are not necessarily 100% bactericidally effective.

A further problem with platinum oxide-catalysed hydrogen peroxide lens cleaning procedures is that the oxygen gas produced during the decomposition reaction can build up within the lens case. The build-up of oxygen is often sufficiently rapid that the cap of the lens case can be blown off. This problem is conventionally solved by the inclusion of vents in the cap to prevent the build-up of pressure. However, in the event that the lens case were knocked over accidentally, then these vents could allow hydrogen peroxide solution to spill.

We have now surprisingly found that the addition of an agent such as glycerol to the hydrogen peroxide solution decreases the rate of the catalytic decomposition of hydrogen peroxide to the extent that the decomposition reaction can be extended, even for periods of up to two hours, or longer.

Thus, in a first aspect, the present invention provides a hydrogen peroxide solution for sterilising contact lenses, characterised in that the solution further contains an agent such as glycerol serving to reduce the overall rate of hydrogen peroxide decomposition in the presence of a suitable catalyst.

Thus, in the presence of an agent such as glycerol, hydrogen peroxide is decomposed more slowly, so that it is cidally effective for longer.

As used herein, "sterilisation" and its related terms refers to killing of micro-organisms present on contact lenses that might, for example, invade the lens or infect the eye. The term "disinfection" is also used in a similar manner herein.

By "agent such as glycerol" is meant any suitable agent that reduces the rate of catalytic decomposition of hydrogen peroxide in the presence of a suitable catalyst, wherein the term "suitable" is used to indicate any catalyst which can be used to catalyse the decomposition of hydrogen peroxide in the presence of the contact lens to be sterilised. A catalyst which disintegrated and/or contaminated the lens in any significant way would not be suitable. Suitable agents will be readily apparent to those skilled in the art, in that they act in a manner similar to glycerol.

While not wishing to be bound by theory, in the case of glycerol, it is believed that the agent affects the catalytic decomposition of hydrogen peroxide in two ways. First, it is likely that glycerol exhibits certain surfactant qualities, in that it allows the hydrogen peroxide solution to more intimately contact more of the surface of the catalyst. Water, for example, will bead on the surface of a metal while, in the presence of even trace quantities of surfactant, it will spread out, and we believe that this is at least partially true for glycerol also On its own, this should result in an even greater production of oxygen. However, we believe that glycerol, in its second mode of action, serves to increase the viscosity of the hydrogen peroxide solution. The increase in viscosity thereby serves to inhibit the rate of exchange at the surface of the catalyst so that, while there is greater availability of catalyst surface area, the rate of contact of hydrogen peroxide with the surface of the catalyst is dramatically reduced.

Thus, by taking the above into consideration, the skilled person will readily be able to establish other suitable agents acting in a manner similar to that of glycerol Although the agent should not be harmful to the lens wearer, even this requirement may be abrogated by washing to remove the agent, for example.

Suitable agents will generally be soluble in water, preferably more than sparingly soluble. Although we currently particularly prefer glycerol, in general terms, it is preferred that the agent is a polyhydric alcohol, preferably a lower polyhydric alcohol. Examples of suitable agents include sorbitol, inositol and polyoxyethylene alcohols such as Tween 20. As referred to herein, "glycerol" should be taken to include any suitable agents, especially those exemplified above.

The combination of the presumed two effects of glycerol results in a slower, more even, release of bubbles. This reduces the vigorous frothing of the hydrogen peroxide as the reaction proceeds, and is manifested in the 'champagne mousse appearance of the solution. During the decomposition of hydrogen peroxide in the presence of glycerol, oxygen gas is produced sufficiently slowly to allow it to escape through the area of contact between the cap and the case, so that it is not necessary to provide the cap with vents. Without vents, the risk of spilling hydrogen peroxide solution is eliminated.

The sterilising solution containing hydrogen peroxide and glycerol will usually be aqueous, preferably being made with high purity de-ionised water. The solution may include other components, such as may, for example, allow for the more efficient sterilisation of the lenses, reduce the violence of bubbling of the hydrogen peroxide while sterilisation proceeds, or to promote increased lens comfort

It will be appreciated that the concentration of hydrogen peroxide used in the sterilising solution may be varied, as is recognised in the art, with the general provision that the concentration be sufficient to sterilise the lenses. A particularly suitable concentration is 3% w/v.

It will also be appreciated that the concentration of glycerol may be varied, provided that sufficient glycerol be present to improve lens sterilisation, but preferably not so much that the cost becomes prohibitive, or that the lens is coated to such an extent that it causes discomfort. In the particular case of glycerol, we generally prefer the concentration range to be 0.5 -10% w/v. More particularly we prefer the range to be 1.5-3.5% w/v, especially 2.5% w/v.

Suitable catalysts generally comprise any material that catalyses the decomposition of hydrogen peroxide, subject to the provisions mentioned above. The catalyst is preferably a solid, and more preferably a metal or metal oxide from Periods 4, 5 or 6 of the Periodic Table, or one of the lanthanide elements. Particularly preferred is platinum, more particularly platinum oxide.

In a preferred embodiment the sterilisation process is carried out so that the final sterilising solution is both buffered and isotonic after the catalytic decomposition of hydrogen peroxide is complete, in order to enhance the wearer's comfort, and suitable means are described in the art.

We have found that the use of contact lenses direct from the sterilising solution in this invention has an additional benefit for the user. The presence of a viscous agent in the solution gives a coating to the contact lenses, which increases the comfort of the contact lens in the eye. However, in the case where the isotonic solution is distinct from the sterilising solution and does not contain a viscous agent, the effect of the agent in enhancing lens comfort may be reduced or lost.

The present invention further provides a method for sterilising contact lenses comprising the use of a solution as described.

Thus, in a further aspect, the present invention provides a method for sterilising contact lenses using a hydrogen peroxide solution in conjunction with a suitable catalyst, characterised in that the hydrogen peroxide solution contains an agent such as glycerol serving to reduce the overall rate of hydrogen peroxide decomposition in the presence of the catalyst.

In the art, sterilisation procedures involve the addition of hydrogen peroxide to a lens case in which the catalyst is already present, the lenses having previously been placed in the case. Thus, sterilisation of the lenses and catalytic decomposition of hydrogen peroxide are initiated simultaneously. In a preferred embodiment, the glycerol is present in the hydrogen peroxide solution at this time, such that it exerts an immediate effect on the catalytic decomposition reaction.

However, it will be appreciated that the glycerol may be added to the case before addition of the sterilisation solution, and that this may serve to reduce the necessary amount of glycerol. This is not generally preferred, as the current optimum concentration of glycerol is 3%, and the addition of glycerol in this manner tends to lead to vastly increased concentrations. Likewise, addition of the glycerol after the reaction has started will tend to have less effect than is desired, so that it is much preferred that the glycerol be present in the sterilisation solution before the reaction commences.

## Claims

1. An hydrogen peroxide solution for sterilising contact lenses, characterised in that the solution further contains an agent such as glycerol serving to reduce the overall rate of hydrogen peroxide decomposition when in the presence of a suitable catalyst.

2. An hydrogen peroxide solution, optionally according to claim 1, for sterilising contact lenses, characterised in that the solution further contains an agent serving to reduce the overall rate of hydrogen peroxide decomposition when in the presence of a suitable catalyst, the agent having surfactant qualities.

3. An hydrogen peroxide solution, optionally according to claim 1 and/or claim 2, for sterilising contact lenses, characterised in that the solution further contains an agent serving to reduce the overall rate of hydrogen peroxide decomposition when in the presence of a suitable catalyst, the agent acting to thicken the solution.

4. A solution according to any preceding claim, wherein the agent is a polyhydric alcohol.

5. A solution according to any preceding claim, wherein the agent is a lower polyhydric alcohol.

6. A solution according to any preceding claim, wherein the agent is one or more of sorbitol, inositol and polyoxyethylene alcohols such as Tween 20.

7. A solution according to any preceding claim, wherein the agent is glcerol.

8. A solution according to any preceding claim, wherein the concentration of hydrogen peroxide is about 3% w/v.

9. A solution according to any preceding claim, wherein the agent is present in a concentration range of from 0.5 -10% w/v.

10. A solution according to any preceding claim, wherein the agent is present in a concentration range of from 1.5-3.5% w/v.

11. A solution according to any preceding claim, wherein the agent is present in a concentration of about 2.5% w/v.

12. A method for sterilising contact lenses using a hydrogen peroxide solution in conjunction with a suitable catalyst, characterised in that the hydrogen peroxide solution is as defined in any preceding claim.

13. A method according to claim 12, which is carried out so that the final sterilising solution is both buffered and isotonic after the catalytic decomposition of hydrogen peroxide is complete.

14. A method according to claim 12 or 13, wherein the agent is present in the hydrogen peroxide solution prior to the solution contacting the catalyst.
